# EUROPEAN PATENT APPLICATION

(11) **EP 4 113 116 A1**
(43) Date of publication of application: **04.01.2023**
(21) Application number: 21186876.5
(22) Date of filing: 21.07.2021
(51) Int. Cl.: G01N 33/14, G01N 21/78, G01N 21/29, G01N 31/22, G01K 11/12, A23F 3/14

(54) **DEVICE AND METHOD FOR INDICATING A CONCENTRATION OF A BEVERAGE**

(30) Priority: 30.06.2021 NL 2028595
(71) Applicant: A.J.M. Tilburg Holding B.V., 6687 LN Angeren (NL); Ron Sloof Holding B.V., 2724 GE Zoetermeer (NL)
(72) Inventor: Sloof, Ronald, 2724 GE Zoetermeer (NL); Tilburg, Adrianus Johannes Maria, 6851 CZ Huissen (NL)
(74) Representative: Arnold & Siedsma

(57) **Abstract**

Indicator device for indicating a concentration of a beverage prepared by extracting at least one compound from a beverage preparation material, such as an infusion or steeping process for the preparation of tea or the like, wherein the indicator device comprises a carrier provided with an optical indicator, wherein the carrier is arranged to be inserted into the beverage for exposing the optical indicator to the beverage, wherein the optical indicator is arranged to indicate whether a concentration of the compound in the beverage corresponds to a predetermined reference concentration of the compound, wherein the compound is a cannabinoid.

## Description

The present invention relates to an indicator device for indicating a concentration of a beverage, for instance tea, prepared by extracting at least one compound from a beverage preparation material. The invention further relates to a steeping system for preparing a beverage by extracting at least one compound from a beverage preparation material, the system comprising such an indicator device. Furthermore, the invention relates to a method for indicating a concentration of a beverage prepared by extracting at least one compound from a beverage preparation material.

Presently, a variety of cannabinoid products, specifically cannabidiol products, are available for consumption. Cannabidiol, a non-psychoactive cannabinoid, may be supplied as an oil containing cannabidiol as an active ingredient. Further available is a cannabidiol tea, an infusion prepared by steeping plant material in water to extract chemical compounds from the material. Strength of the tea is determined intuitively on the basis of the amount of time the material is kept in the water. A problem with e.g. cannabidiol tea is that with conventional preparation methods the dose of the cannabinoid cannot be sufficiently controlled but is largely dependent on the preparation material.

It is therefore a goal, amongst other goals, of the invention to enable a consumer of a beverage containing a compound, particularly a cannabinoid tea, to determine, or at least to receive an indication of, the dosage of the compound in a reliable and/or user-friendly manner.

This goal, amongst other goals, is met by an indicator device for indicating a concentration of a beverage prepared by extracting at least one compound, preferably a cannabinoid, from a beverage preparation material, such as an infusion or steeping process for the preparation of tea or the like. The indicator device comprises a carrier provided with an optical indicator. The carrier is arranged to be inserted into the beverage for exposing the optical indicator to the beverage. The optical indicator is arranged to indicate whether a concentration of the compound in the beverage corresponds to a predetermined reference concentration of the compound.

Instead of requiring professional testing for determining specifically the concentration of the compound, the optical indicator making use of the predetermined reference concentration enables a consumer of the beverage to steep the beverage preparation material until a desired concentration of the compound is obtained, which is when the optical indicator provides an indication that the concentration corresponds to the predetermined reference concentration of the compound. For instance, the optical indicator may be exposed to water at the start of the steeping process for preparing the beverage. The compound is extracted from the beverage preparation material, whereby the concentration of the compound in the beverage increases. When a concentration corresponding to the predetermined reference concentration is reached, the optical indicator provides an indication. As such, the indicator device can attribute a value to the dose of a cannabinoid in tea in a convenient manner.

The beverage may be e.g. a mixture containing one or more chemical compounds extracted from the beverage preparation ingredient in hot water, specifically water at a near-boiling temperature, to prepare the beverage mixture. The carrier may be a paper-like strip or stick of synthetic material.

Although the above problem is described in the context of a cannabinoid beverage, it is to be appreciated that the compound may be any other compound, such as caffeine or a flavouring agent, for which it may be preferable if determining a concentration thereof is enabled in a similar manner as described above.

According to an embodiment of the indicator device, the optical indicator comprises at least one chromic material. The chromic material is preferably arranged to vary an optical property thereof, such as intensity, which can be defined as luminance, and/or colour or any other suitable optical property. The optical property is then preferably related to the predetermined reference concentration of the compound for determining the concentration of the compound. This way, the optical property can be directly or indirectly related to the concentration of the compound. As such, the concentration of the compound can be determined on the basis of the optical property. More specifically, the concentration of the compound may be determined by relating the optical property to at least one predetermined optical reference property.

In case the optical property is related to a predetermined optical reference property, it is preferred if the indicator device further comprises at least one reference marking indicating the at least one predetermined optical reference property. The reference marking, e.g. a coloured marking, enables the optical property to be directly compared with the predetermined optical reference property. When the colour of the optical indicator shifts to the colour of the reference marking, it can be easily detected whether the concentration of the compound is equal, or approximately equal, to the predetermined reference concentration. For instance, a segment of the carrier that is to be kept out of the beverage may be provided with the reference marking to enable an easy comparison. Preferably, the at least one reference marking comprises an optical reference scale. The optical reference scale preferably corresponds to a plurality of predetermined reference values, each representative for a predetermined reference concentration of the compound.

The chromic material may for example comprise a chronochromic material. However, according to a preferred embodiment of the indicator device the chromic material comprises a reactant for chemically reacting with at least one compound, preferably the cannabinoid, for varying the optical property. The reactant preferably comprises at least one of an enzyme, an alga, a pigment and potassium hydroxide. The reactant may therewith be considered a concentratochromic material, preferably arranged to vary an optical property thereof in dependency of the concentration of the compound.

A chromic material can thus be defined herein as any material changing colour and/or intensity or any like optical property under the influence of a determinant directly related to the concentration of the compound, e.g. in the case of a reactant for chemically reacting with the compound, or indirectly as in the example of a further preferred embodiment of the indicator device described hereafter. According to this preferred embodiment, the chromic material comprises a thermochromic material. The thermochromic material is preferably arranged to vary a colour thereof, preferably in dependency of a temperature of the beverage. Since extraction of a compound may be in part dependent on a temperature of the solvent, the concentration of the compound in the solvent can be consequently related to the temperature thereof. The thermochromic material can thus be arranged to change colour under the influence of a circumstantial factor, in this particular case a temperature of the beverage, indirectly related to the concentration of the compound. In other words, the concentration of the compound can be correlated with the temperature of the beverage.

To accurately indicate the concentration, the optical indicator is preferably calibrated with respect to the circumstantial factor, which is the beverage temperature in the case of the thermochromic material. It is then to be predetermined how the compound extraction process progresses from the moment the beverage preparation material is steeped in water at a predetermined starting temperature, in order to be able to predetermine an appropriate reference concentration for the optical indicator at a different temperature. If the optical property of the indicator shifts at e.g. 80 degrees Celsius and the starting temperature is set at 100 degrees Celsius, the reference concentration can then be determined to equal the concentration of the compound measured at 80 degrees Celsius for a beverage prepared with a starting temperature of 100 degrees Celsius.

According to another example of a further preferred embodiment of the indicator device, the chromic material comprises a halochromic material, preferably arranged to vary a colour thereof, preferably in dependency of an acidity or basicity of the beverage. Along with extraction of a compound, a pH value of the solvent may change. Hence, the concentration of the compound can be related to the acidity or basicity of the solvent. The halochromic material can thus be arranged to change colour under the influence of the acidity or basicity of the beverage indirectly related to the concentration of the compound in a similar manner as the thermochromic can change colour in correlation with the concentration of the compound.

According to a further embodiment of the indicator device, the optical indicator is arranged to form an optical contrast with the beverage. The optical contrast may be formed, at least on the part of the optical indicator, by any optical property of the optical indicator as described above. Additionally or alternatively, the contrast may be formed, at least on the part of the beverage, by an optical property of the beverage, such as colour and/or transparency, which is variable under the influence of a factor directly or indirectly related to the concentration of the compound. The optical contrast can thus be formed in correlation with the concentration of the compound in the beverage. Preferably, the optical contrast is related to at least one predetermined reference contrast corresponding to at least one predetermined reference concentration of the compound. This way, the optical contrast can be related to the concentration of the compound via the reference contrast and/or the reference concentration. As such, the concentration of the compound can be determined on the basis of the optical contrast.

According to a further embodiment of the indicator device, the optical indicator comprises at least one optical marking having an optical property such as intensity and/or colour. The optical marking may be arranged for forming, at least on the part of the optical indicator, the optical contrast as described above. The optical marking, in particular the optical property thereof, is preferably related to the predetermined reference concentration of the compound for determining the concentration of the compound. As such, the concentration of the compound can be determined on the basis of the optical property of the optical marking. For example, the optical marking may have a colour similar to a colour of the beverage having reached the predetermined reference concentration of the compound in the beverage, such that the concentration of the compound can be determined to be equal to the predetermined reference concentration. The reference concentration can be determined by calibrating the optical indicator with respect to the changing optical property of the beverage in correlation with the concentration of the compound in a similar manner as in the case of the thermochromic material, as described above.

Preferably, the at least one optical marking comprises an optical scale. The optical scale preferably corresponds to a plurality of predetermined reference values. It is then further preferred if each predetermined reference value is representative for a predetermined reference concentration of the compound. This way, a relatively accurate assessment of the dosage of the compound in the beverage can be made for both a relatively high dose and a relatively low dose.

According to another aspect is provided a steeping system for preparing a beverage by extracting at least one compound from a beverage preparation material, such as an infusion or steeping process for the preparation of tea or the like, wherein the steeping system comprises a water permeable pouch for containing the beverage preparation material, and wherein the steeping system comprises an indicator device according to any one of the above embodiments. The indicator device may then be specifically adapted to the steeping system, in particular to the beverage preparation material, for providing an accurate indication of the concentration of the compound in the beverage. The pouch may be a sachet or a stick, similar to a tea stick. Such a stick may comprise a sealing layer, made for instance of a synthetic material such as plastic, provided with perforations to enable a beverage preparation material held therein to come into contact with a solvent for extracting a compound from the beverage preparation material. The pouch may also comprise a cellulose material such as abaca fibre or the like, preferably a water-resistant and water-permeable material, or any other paper-like material. Since the beverage is for human consumption purposes, it is further preferred if the material does not stain the taste or smell of the beverage.

Preferably, the optical indicator is provided on the pouch. This way, it can be ensured that the optical indicator is exposed to the beverage from the start of the extraction or steeping process, which is particularly advantageous in case the optical indicator comprises a chronochromic material or a reactant of which the reaction, and therewith the optical property of the optical indicator, is dependent on the amount of time the reactant is exposed to the compound.

An embodiment of the steeping system further comprises a packet enclosing the pouch and preferably the optical indicator. As such, the pouch and the optical indicator can be packaged together so as to be covered from external factors affecting e.g. the performance of the optical indicator. More in particular, oxidation of the compounds, in for instance the beverage preparation material or the optical indicator, can be prevented. A compact, user-friendly beverage preparation product for preparing a tea with a precisely obtainable cannabinoid dose, as desired, can thus be provided. In an alternative embodiment, the optical indicator may be provided on the packet.

A further embodiment of the steeping system comprises the beverage preparation material. Preferably, the pouch contains the beverage preparation material.

According to yet another aspect, a kit of parts is provided, comprising a water permeable pouch for containing a beverage preparation material for preparing a beverage by extracting at least one compound from the beverage preparation material, such as an infusion or steeping process for the preparation of tea or the like, and a separate indicator device according to any one of the above embodiments.

Further provided is a method for indicating a concentration of a beverage prepared by extracting at least one compound from a beverage preparation material, such as an infusion or steeping process for the preparation of tea or the like, wherein the compound is preferably a cannabinoid. The method comprises the steps of providing the beverage; providing an indicator device, preferably according to any one of the above embodiments, comprising a carrier provided with an optical indicator for indicating whether a concentration of the compound in the beverage corresponds to a predetermined reference concentration of the compound; determining an optical indication of the optical indicator; determining the concentration of the compound on the basis of the determined optical indication and the predetermined reference concentration of the compound.

Preferably, the step of providing the beverage comprises the step of extracting the compound from the beverage preparation material. It is then further preferred if the step of providing the indicator device comprises providing the optical indicator in contact with the beverage simultaneously with the step of extracting the compound from the beverage preparation material. This way, the optical indicator can be exposed to the beverage from the start of the extraction or steeping process.

Further, the step of determining the concentration of the compound preferably comprises relating the determined optical indication to at least one predetermined optical reference indication corresponding to the at least one predetermined reference concentration.

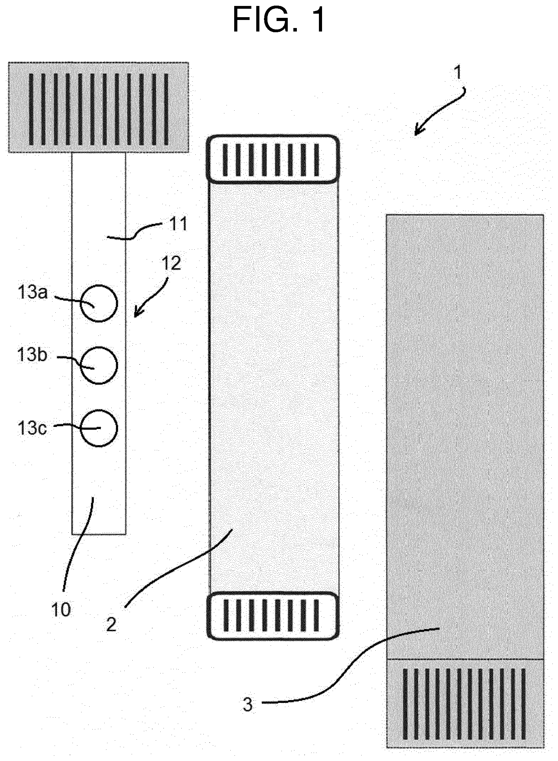

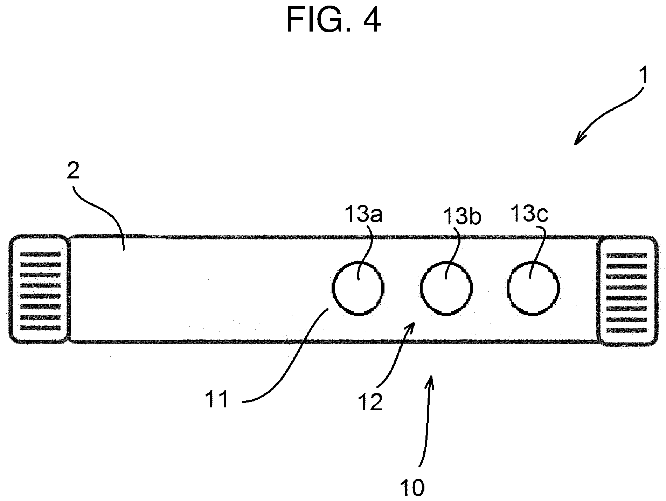

The invention is hereafter further elucidated with reference to the appended drawings, wherein figures 1-5 show various embodiments of a steeping system with an indicator device. Throughout the figures, like elements are referred to using like reference numerals.

In figures 1-5 a steeping system 1 is schematically depicted. The steeping system 1 comprises a water permeable pouch 2 containing tea leaves for preparing a cannabidiol tea. Other beverage preparation material may be contained by the pouch 2 as further alternatives. By immersing the pouch 2 containing the leaves in hot water, the water can permeate through the pouch 2 for extracting a cannabinoid compound from the leaves. With such an infusion or steeping process, cannabidiol tea or the like can be prepared.

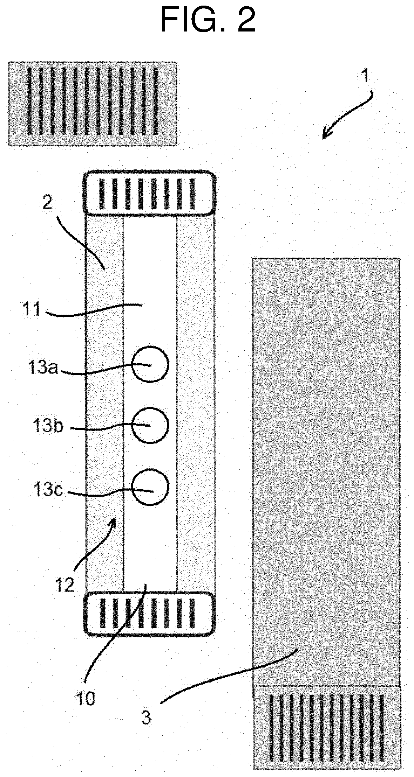

The steeping system 1 further comprises an indicator device 10 comprising a carrier 11, in this example in the form of a strip shaped member, provided with an optical indicator 12. Although in the example shown in figure 1 the optical indicator 12 is separate from the pouch 2, the optical indicator 12 in figures 2-5 is provided on the pouch 2 to be automatically exposed to the tea for the duration of the extraction process for indicating whether a concentration of the cannabinoid in the tea corresponds to a predetermined reference concentration of the compound. Thereto, the optical indicator 12 in figures 1, 2 and 4 comprises an optical scale. The scale comprises three optical markings 13a, 13b, 13c with different optical properties, e.g. colour. The optical markings 13a, 13b, 13c correspond to three predetermined reference values, each representative for a predetermined reference concentration of the compound.

During the steeping process, an optical property of the tea may vary in dependency of the duration of the extraction process. In this example, the tea turns darker over time. Once the colour of the tea approximates the colour of one of the optical markings 13a, it can be deduced that the concentration of the cannabinoid in the tea approximates the reference value related to that one optical marking 13a. Similarly, it can e.g. be deduced that the concentration of the cannabinoid in the tea is below the reference value as long as the colour of the tea is brighter than the colour of that one optical marking 13a. Furthermore, the optical indicator 12 may form an optical contrast with the tea, wherein the optical contrast is related to at least one predetermined reference contrast corresponding to the predetermined reference concentration of the compound.

The optical property of the optical indicator 12 thus has to be predetermined, or calibrated, for instance on the basis of experiments for identifying the colour of the tea at the predetermined reference concentration of the compound in the tea. This way, the colour of the tea, which changes during the preparation of the tea and which can be conveniently detected, can function as a proxy for the concentration of the cannabinoid or other compound in the beverage.

The steeping system 1 shown in figures 1-3 further comprises a packet 3 for enclosing the pouch 2 and the indicator device 10. In figures 4 and 5, the pouch 2 containing the tea leaves is formed as a tea stick.

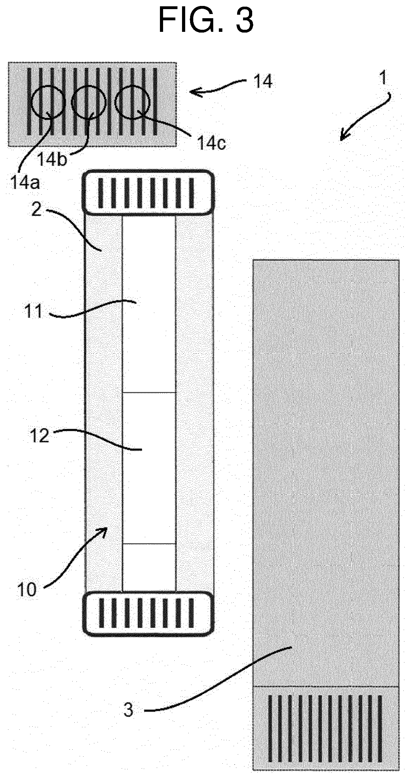

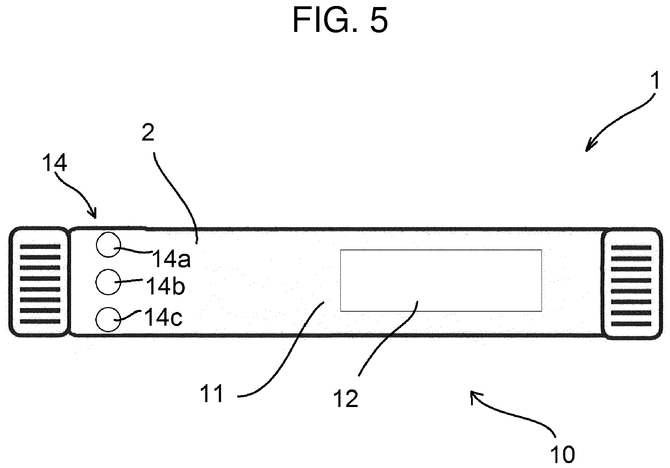

Figures 3 and 5 show other examples of a steeping system 1 with an indicator device 10. The optical indicator 12 of the indicator device 10 comprises a chromic material for varying an optical property thereof. The optical property is related to the predetermined reference concentration of the cannabinoid for determining the concentration of the cannabinoid in the tea. The chromic material may comprise a reactant for chemically reacting with the cannabinoid for varying the optical property. It is however also possible that the reactant is to react with another compound in the beverage, of which the concentration in the beverage can function as a proxy for the concentration of the cannabinoid.

The indicator device 10 further comprises an optical reference scale 14. The scale 14 comprises three reference markings 14a, 14b, 14c with different optical properties, e.g. colour. The reference markings 14a, 14b, 14c indicate three predetermined optical reference properties, or colours, to which the optical property of the optical indicator 12 can be related. Each reference property represents a predetermined reference concentration of the compound. For instance, once the colour of the chromic material approximates the colour of one of the reference markings 14a, it can be deduced that the concentration of the cannabinoid in the tea approximates the reference value related to that one reference marking 14a. The scale 14 is arranged at a part of the steeping system 1 that is to be kept out of the beverage, for instance at an end of the carrier 11, as shown in figures 5, or on the packet 4.

The chromic material may comprise a thermochromic material for varying an optical property thereof in dependency of a temperature of the tea. During the steeping process, the temperature of the tea may lower in dependency of the duration of the extraction process as the tea cools down due to heat loss to the ambient. Since the temperature of the tea can thus be related to the duration of the extraction process which in turn can be related to the concentration of the cannabinoid in the tea, the concentration of the cannabinoid in the tea can be deduced from the optical property of the thermochromic material. For this, the optical property of the chromic material has to be calibrated, for instance on the basis of experiments for identifying the temperature of the tea at the predetermined reference concentration of the compound in the tea. This way, the temperature of the tea, which changes during the preparation of the tea and which can be conveniently detected using e.g. the thermochromic material, can function as a proxy for the concentration of the cannabinoid or other compound in the beverage. It is then preferred if the starting temperature of the steeping process is equal to the starting temperature set for the experiments, for the optical property of the chromic material to accurately represent the concentration of the compound.

Similarly, the chromic material may comprise a halochromic material for varying an optical property thereof in dependency of an acidity or basicity of the beverage. During the steeping process, the pH value of the tea may lower in dependency of the duration of the extraction process. Since the acidity of the tea can thus be related to the duration of the extraction process, the concentration of the cannabinoid in the tea can be deduced from the optical property of the halochromic material. In other words, the pH value of the tea, which changes during the preparation of the tea and which can be conveniently detected using e.g. the halochromic material, can function as a proxy for the concentration of the cannabinoid or other compound in the beverage.

More generally, the optical indicator 12 may be configured for varying an optical property thereof in dependency of any conveniently detectable proxy of the beverage that is representative for the concentration of the compound in the beverage.

Whilst the principles of the present invention have been set out above in connection with specific embodiments, it is to be understood that this description is merely made by way of example and not as a limitation of the scope of protection which is determined by the appended claims.

## Claims

1. Indicator device for indicating a concentration of a beverage prepared by extracting at least one compound from a beverage preparation material, such as an infusion or steeping process for the preparation of tea or the like, wherein the indicator device comprises a carrier provided with an optical indicator, wherein the carrier is arranged to be inserted into the beverage for exposing the optical indicator to the beverage, wherein the optical indicator is arranged to indicate whether a concentration of the compound in the beverage corresponds to a predetermined reference concentration of the compound, wherein the compound is a cannabinoid.

2. Indicator device according to claim 1, wherein the optical indicator comprises a chromic material which is arranged to vary an optical property thereof, such as intensity and/or colour, wherein the optical property is related to the predetermined reference concentration of the compound for determining the concentration of the compound.

3. Indicator according to claim 2, wherein the chromic material comprises a reactant for chemically reacting with the compound for varying the optical property.

4. Indicator device according to claim 3, wherein the reactant comprises an enzyme, an alga, a pigment or potassium hydroxide.

5. Indicator device according to claim 2, 3 or 4, wherein the chromic material comprises a thermochromic material, wherein the thermochromic material is arranged to vary a colour and/or intensity thereof in dependency of a temperature of the beverage.

6. Indicator device according to any of the preceding claims 2 - 5, wherein the chromic material comprises a halochromic material, wherein the halochromic material is arranged to vary a colour and/or intensity thereof in dependency of an acidity or basicity of the beverage.

7. Indicator device according to any of the preceding claims 2 - 6, wherein the optical property is related to at least one predetermined optical reference property, wherein the indicator device further comprises at least one reference marking indicating the at least one predetermined optical reference property.

8. Indicator device according to any of the preceding claims, wherein the optical indicator is arranged to form an optical contrast with the beverage, wherein the optical contrast is related to at least one predetermined reference contrast corresponding to at least one predetermined reference concentration of the compound.

9. Indicator device according to any of the preceding claims, wherein the optical indicator comprises at least one optical marking having an optical property such as intensity and/or colour, wherein the optical marking is related to the predetermined reference concentration of the compound for determining the concentration of the compound.

10. Steeping system for preparing a beverage by extracting at least one compound from a beverage preparation material, such as an infusion or steeping process for the preparation of tea or the like, wherein the steeping system comprises a water permeable pouch for containing the beverage preparation material, wherein the steeping system comprises an indicator device according to any one of the preceding claims 1-9.

11. Steeping system according to claim 10, wherein the optical indicator is provided on the pouch.

12. Steeping system according to claim 10 or 11, further comprising a packet enclosing the pouch and the optical indicator.

13. Kit of parts comprising a water permeable pouch for containing a beverage preparation material for preparing a beverage by extracting at least one compound from the beverage preparation material, such as an infusion or steeping process for the preparation of tea or the like, and a separate indicator device according to any one of the preceding claims 1-9.

14. Method for indicating a concentration of a beverage prepared by extracting at least one compound from a beverage preparation material, such as an infusion or steeping process for the preparation of tea or the like, wherein the compound is a cannabinoid, wherein the method comprises the steps of:
- providing the beverage;
- providing an indicator device comprising a carrier provided with an optical indicator for indicating whether a concentration of the compound in the beverage corresponds to a predetermined reference concentration of the compound;
- determining an optical indication of the optical indicator;
- determining the concentration of the compound on the basis of the determined optical indication and the predetermined reference concentration of the compound.

15. Method according to claim 14, wherein the step of providing the beverage comprises the step of extracting the compound from the beverage preparation material, wherein the step of providing the indicator device comprises providing the indicator device in contact with the beverage simultaneously with the step of extracting the compound from the beverage preparation material.
